(19) Europäisches Patentamt
European Patent Office
Office européen des brevets

(11) Publication number: **0 407 037 B1**

(12) **EUROPEAN PATENT SPECIFICATION**

(45) Date of publication of patent specification: **21.09.94**

(51) Int. Cl.5: **A61K 39/02**, A61K 39/102

(21) Application number: **90306237.0**

(22) Date of filing: **08.06.90**

(54) **Process for removing bacterial endotoxin from gram-negative polysaccharides.**

(30) Priority: **12.06.89 US 364929**
**12.06.89 US 364911**
**30.11.89 US 443024**
**12.06.89 US 364927**

(43) Date of publication of application:
**09.01.91 Bulletin 91/02**

(45) Publication of the grant of the patent:
**21.09.94 Bulletin 94/38**

(84) Designated Contracting States:
**CH DE FR GB IT LI NL**

(56) References cited:
**EP-A- 0 072 513**
**EP-A- 0 145 359**
**US-A- 3 978 209**
**EP-A- 0 117 783**
**US-A- 3 636 192**
**US-A- 4 307 080**

(73) Proprietor: **MERCK & CO. INC.**
**126, East Lincoln Avenue**
**P.O. Box 2000**
**Rahway New Jersey 07065-0900 (US)**

(72) Inventor: **Rienstra, Mark S.**
**405 Bonnie Lane**
**Lansdale, PA 19446 (US)**
Inventor: **Scattergood, Edgar M.**
**1375 Stevenlane**
**Lansdale, PA 19446 (US)**
Inventor: **Sitrin, Robert D.**
**237 Emerson Drive**
**Lafayette Hill, PA 19444 (US)**

(74) Representative: **Cole, William Gwyn et al**
**European Patent Department**
**Merck & Co., Inc.**
**Terlings Park**
**Eastwick Road**
**Harlow Essex CM20 2OR (GB)**

**EP 0 407 037 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

The invention is a process for removing bacterial endotoxin from Gram-negative polysaccharides without incurring substantial loss of polysaccharide.

Bacterial endotoxin is a potent pyrogen that can often produce fever reactions when administered to patients. Endotoxin is an integral component of the outer cell surface of Gram-negative bacteria. It exists in its natural state as a complex of lipid, carbohydrate and protein. When highly purified, endotoxin does not contain protein, and by its chemical composition is referred to as a lipopolysaccharide (see Weary and Pearson, Bio. Pharm. April (1988) pp. 22-29).

The outer-wall layer of Gram-negative bacteria serves as an outer barrier through which materials must penetrate if they are to reach the cell. It is selectively permeable. Generally, endotoxin is released in large amounts only when the cell wall is lysed.

Removal of contaminating endotoxin from Gram-negative polysaccharides is important when the polysaccharide is to be administered to humans. Endotoxins in large quantities can cause shock, severe diarrhea, fever and leukopenia followed by leukocytosis, and can elicit the Shwartzman and Sanarelli-Shwartzman phenomena.

In US-A-4307080 there is described a vaccine effective against Hemophilus influenzae type b, comprising a high molecular weight capsular polysaccharide from H. influenzae type b, which is stated to be an effective immunogenic agent against infection caused by H. influenzae bacterium.

EP-A-0117783 also describes and claims a vaccine effective against H. influenzae type b, which is based on a polysaccharide/protein conjugate and stated to have enhanced antigenicity and immunogenicity relative to the unconjugated polysaccharide. The conjugate comprises H. influenzae type b polysaccharide and a T-cell stimulating Neisseria meningitidis serotype outer protein, the polysaccharide and protein being coupled through 6-aminocaproic acid.

United States Patent No. 4,695,624, issued to Marburg et al., describes covalently-modified polyanionic bacterial polysaccharides, stable covalent conjugates of these polysaccharides with immunogenic proteins, and methods of preparing the polysaccharides and conjugates and of confirming covalency. The patent describes purification of the polysaccharide in Example 1, beginning in column 14. After fermentation, inactivation and cell removal, the resulting product undergoes a series of cold ethanol fractionations. Following phenol extraction are diafiltration, ethanol precipitation, ultracentrifugation in ethanol, and collection of the finished product.

Frequently, the amount of contaminating endotoxin remaining after the above-described procedure is higher then desired.

Methods for removing endotoxin which are known in the art are described by Weary and Pearson (ibid): rinsing with nonpyrogenic solution (Feldstine et al., J. Parenter. Drug Assoc., 33, p. 125 (1979) and Berman et al., J. Parenter. Sci. Technol., 41, p. 158 (1987); distillation; ultrafiltration using membranes rated by molecular weight exclusion (Sweadner et al., Appl. Environ. Microbiol., 34, p. 382 (1977) and Henderson et al., Kidney Int., 14, p. 522 (1978); reverse osmosis using thin cellulose acetate or polyamide materials (Nelson, Pharm. Technol., 2, p. 46 (1978); electrostatic attraction (Gerba et al., Pharm Technol., 4, p. 83 (1980) and Hou et al., Appl. Environ. Microbiol., 40, p. 892 (1980); hydrophobic attraction using aliphatic polymers (Robinson et al., in Depyrogenation (Parental Drug Association, Philadelphia (1985), pp. 54-69); adsorption using activated carbon (Berger et al., Adv. Chem. Ser., 16, p. 169 (1956), Gemmell et al., Pharm J., 154, p. 126 (1945), and Brindle et al., Pharm J., 157, p. 85 (1946); and affinity chromatography (Soter, Bio/Technology, 12, p. 1035 (1984).

Sawada, et al., Applied and Environmental Microbiology, April 1986, pp. 813-820, describe removal of endotoxin from water by microfiltration through a microporous polyethylene hollow-fiber membrane. Gerba et al., Applied and Environmental Microbiology, December 1985, pp. 1375-1377, describe endotoxin removal from various solutions using charged nylon and cellulose-diatomaceous earth filters. Nolan et al., Proceedings of the Society for Experimental Biology and Medicine, Vol. 149, pp. 766-770 (1975), describe endotoxin binding by charged and uncharged resins.

Sweadner, K. et al., Applied and Environmental Microbiology, Vol. 34, pp. 382-385 (1977) explain that lipopolysaccharide often exist in an aggregated state, and that dissociating the lipopolysaccharide with detergent or chelating agents can facilitate its removal from aqueous solutions by filtration. Shands, J. et al., J. Biological Chemistry, Vol. 255, pp. 1221-1226, show that lipopolysaccharide is associated with divalent cations, and that dispersion of Gram-negative lipopolysaccharides can be achieved using deoxycholate.

McIntire, et al, Biochemistry, Vol. 8, No. 10, pp. 4063-4066 (1969) describes reversible inactivation, by sodium deoxycholate, of Escherichia coli lipopolysaccharide. Ribi, et al., Journal of Bacteriology, Vol. 92, No. 5, pp. 1493-1509 (1966) described physical and biological properties of endotoxin treated with sodium deoxycholate.

It is a purpose of the present invention to provide an effective method of obtaining Gram-negative polysaccharide mixtures having low or negligible levels of endotoxin, without suffering substantial loss of polysaccharide.

It is also a purpose of the present invention to provide a process for eliminating endotoxin from a solution of bacterial polysaccharide while minimizing the removal of bacterial polysaccharides and other desired species.

## SUMMARY OF THE INVENTION

The invention provides, in one aspect, a process for removing endotoxin from a Gram-negative fermentation product, including polysaccharides such as polyribosylribitol phosphate (PRP), which comprises the steps of:

(a) growing Gram-negative bacteria in fermentation broth, releasing polysaccharide into the broth, and adding alcohol (e.g. ethanol) to the broth to remove impurities by precipitation;

(b) isolating the remaining high molecular weight species and dissolving them in calcium chloride solution, removing additional impurities by completing a minimum of one additional alcohol fractionation, collecting and drying the second alcohol precipitate, dissolving the dry powder thereby obtained in sodium acetate and extracting with phenol to remove impurities, and subsequently removing the phenol from the aqueous solution containing polysaccharide by diafiltration with water;

(c) centrifuging remaining high molecular weight species and resolubilizing in a counter ion solution; and

(d) adding alcohol to the solution, cooling the solution and thereafter incrementally adding alcohol to achieve lipopolysaccharide/polysaccharide precipitation by selective alcohol fractionation;

characterised in that it also comprises the following step:

(e) mixing lipopolysaccharide-and polysaccharide-containing material resulting from the alcohol addition with a nonionic resin, a detergent and a chelating agent, under basic pH, to remove lipopolysaccharide by resin elimination.

Preferably, the initial addition of alcohol and the temperature after cooling in step (d) results in an alcohol concentration which is up to 2%, preferably between 0.5-1% below the alcohol concentration at the cloud point. Incremental alcohol addition is preferably a sequential addition of about 0.2% at a time until a two-fold increase in turbidity occurs, at which time the cloud point has been reached. The cloud point is the percentage of alcohol when endotoxin and polysaccharide start to precipitate, causing turbidity. After the cloud point has been reached, an additional amount of alcohol is added which results in precipitation of most of the endotoxin with some polysaccharide and a negligible amount of endotoxin remaining in solution.

The counter ion is preferably divalent, but may be monovalent.

Various alcohols may be successfully used during endotoxin removal. Suitable alcohols include denatured ethanol (SDA3A, which is 4.7% MeOH, 88.1% EtOH, 7.2% $H_2O$), 95% EtOH, absolute EtOH, isopropanol, and other alcohols having 1 to 4 carbons which precipitate endotoxin.

Material mixed with resin, detergent and chelating agent may be powder derived from the operation in step (d). Such powder is obtained by drying the precipitate resulting from step (d). It is comprised of polysaccharide and lipopolysaccharide.

Material mixed with resin, detergent and chelating agent may also be a solution obtained from the operation in step (d) which comprises polysaccharide and lipopolysaccharide.

Subjecting these materials to treatment with resin, detergent and chelating agent removes substantially all lipopolysaccharide and improves the overall yield of purified polysaccharide which would otherwise be obtained using only incremental alcohol fractionation. Furthermore, use of the resin elimination methodology allows for manipulation of the amounts of non-polysaccharide and non-lipopolysaccharide species in the finished product, while achieving endotoxin removal.

After treatment of the material in step (e), the resin is removed and the polysaccharide precipitated from solution with alcohol. The precipitate is centrifuged, the pellet triturated with alcohol and the resulting product dried to form a powder.

Removal of lipopolysaccharides by the process of selective alcohol fractionation in combination with resin elimination is useful for manipulating the amounts of materials other than polysaccharides and lipopolysaccharides in the final product. Selective alcohol fractionation removes materials primarily on the basis of molecular weight. Increasing concentrations of alcohol result in elimination of species of decreasing

molecular weight. Removal of lipopolysaccharide using resin depends on the ability of the resin to recognize lipopolysaccharide structures and eliminate species of that nature from solution. Therefore, the process of the present invention is particularly advantageous for minimizing undesirable eliminations of components other than polysaccharides and lipopolysaccharides. Amounts of components other than polysaccharides and lipopolysacchardes can be regulated by utilizing either the selective alcohol fractionation or resin elimination to a greater extent while obtaining the desirable result of essentially eliminating endotoxin from the final product.

In another aspect, the invention provides a method for removing endotoxins from the outer cell of Gram-negative bacteria which comprises the steps of:

(a) growing Gram-negative bacteria in fermentation broth, releasing polysaccharide into the broth, and adding alcohol to remove impurities by precipitation;

(b) isolating the remaining high molecular weight species and dissolving them in calcium chloride solution, removing additional impurities by completing a minimum of one additional alcohol fractionation, collecting and drying the second alcohol precipitated dissolving the dry powder thereby obtained in sodium acetate and extracting with phenol to remove impurities, and subsequently removing the phenol from the aqueous solution containing polysaccharide by diafiltration with water; and

(c) precipitating and removing impurities by adding ethanol to the product of (b);

characterised in that it also comprises the following steps:

(d) drying the resulting solution of (c), and dissolving the resulting powder in a solution of chelating agent and detergent under basic pH and mixing nonionic resin under suitable conditions; and

(e) removing the resin, chelating agent and detergent and precipitating the polysaccharide from solution with ethanol, centrifuging the precipitate, triturating the pellet with ethanol, and drying the resulting product to form a powder.

The following abbreviations are used in the description of the present invention:

| | |
|---|---|
| PRP - | polyribosylribitol phosphate, an H.influenzae type b capsular polysaccharide. |
| LAL test value - | limulus ameobocyte lysate test value, which is an indication of endotoxin level in the end-product. |
| LPS - | lipopolysaccharide, which is the general structure of endotoxin when it is apart from the outer cell surface of Gram-negative bacteria. |
| EU/μg - | Endotoxin units (a measure of LPS) per microgram PRP. |

## DETAILED DESCRIPTION OF THE INVENTION

In one embodiment of the invention, a production fermenter containing complete Haemophilus medium with an antifoaming agent is inoculated with the seed culture. The fermenter is maintained at 37 ± 3 °C for a minimum of twelve hours with moderate aeration and agitation. The H. influenzae type b culture is inactivated after the fermentation is completed by addition of thimerosal under agitation. Cell debris, media components and other impurities are removed by centrifugation or filtration and discarded. The culture supernatant is concentrated by ultrafiltration and additional impurities are removed by alcohol fractionation.

The high molecular weight species are dissolved in calcium chloride solution and a minimum of one additional alcohol fractionation is completed as described above to remove additional impurities. The second alcohol precipitate is collected by centrifugation and a dry powder is obtained by resuspending the precipitate in absolute ethanol followed by filtration, acetone wash and drying.

The powder is dissolved in sodium acetate solution and extracted several times with phenol to remove impurities. The aqueous solution containing polysaccharide is diafiltered with water to remove phenol. Calcium chloride solution is added to the solution and high molecular weight species are precipitated with alcohol and collected by centrifugation. The post phenol powder is resolubilized in calcium chloride solution and is then subjected to selective alcohol fractionation.

Selective alcohol fractionation is an effective process for reducing the level of endotoxin to the point where it meets product specification, while minimizing the loss of polysaccharide from solution. By changing the alcohol concentration, different molecular weight species become insoluble and precipitate out of solution. Increasing alcohol concentration precipitates species of decreasing molecular weight. Ethanol is incrementally added, thereby increasing ethanol concentration towards the cloud point. When the cloud point is reached, polysaccharide and LPS precipitate. Because it can be advantageous to recover the polysaccharide precipitating with the lipopolysaccharide, the combination is dried to a "low cut" powder and later treated by the resin elimination method. Lipopolysaccharide is precipitated along with some polysaccharide, leaving polysaccharide in solution essentially unaccompanied by lipopolysaccharide.

The precipitating material which contains lipopolysaccharides, polysaccharides and other species, such as proteins and lipids, is combined with a resin, a detergent and a chelating agent to remove polysaccharide. The material is first combined with the detergent and chelating agent under basic pH, and resin beads are then added to and mixed with the solution in an orbital shaker for several hours below room temperature. The beads are then removed from solution, and the filtrate is diafiltered using hollow fiber membranes to remove detergent and chelating agent. Retentate is recovered and calcium chloride added. The polysaccharide is precipitated from solution with alcohol. The precipitate is centrifuged and the pellet is triturated with alcohol and acetone. The resulting product is vacuum dried. This process reduces endotoxin level without significant loss of polysaccharide.

The process of the present invention, therefore, accomplishes removal of impurities such as lipopolysaccharides from fermentation products of Gram-negative bacteria by selective alcohol fractionation followed by treatment with resin, a detergent and a chelating agent.

Although sodium citrate is a preferred chelating agent, other chelating agents which are capable of acting on divalent calcium ions present in the solution, and which are capable of serving as a buffer for maintaining basic pH are suitable. Other suitable chelating agents include ethylenediaminetetraacetic acids such as disodium ethylenediaminetetraacetic acid. Preferably, the amount of chelating agent is between about 1% and about 10%, more preferably between about 2% and about 7% and even more preferably about 6%.

Although deoxycholate is a preferred detergent, other detergents which are capable of breaking aggregated lipopolysaccharide are suitable. Suitable detergents include Triton X-100, CHAPS, sodium dodecyl sulfate, and sodium lauryl sulfate. Preferably, the amount of detergent is between about 0.1% and about 2.0%, more preferably between about 0.2% and about 1.0% and even more preferably about 0.75%.

Nonionic resins which bind to lipopolysaccharide, which do not bind to polysaccharides, and which are useful in the present invention include but are not limited to Borate Avidgel® (Amicon), Amberlite XAD® and Amberchrome® (Rohm & Haas), Octyl Cellulose® (Phoenix Chem.), Silicon C8® (Baker), SP207® and HP20® (Mitsubishi Chem.). Of these resins, HP20® is preferred because of lipopolysaccharide reduction, ease of use, availability, cost, and its propensity to avoid binding to polysaccharides. Preferably the resin is washed prior to use with pyrogen free water. More preferably, the resin is washed prior to use with acid solution, an alkali solution, or a polar solvent (e.g. ethanol or methanol) and then with pyrogen free water.

In another embodiment of the invention, powder obtained by drying the precipitate resulting from step (h), which comprises H. influenzae type b bacterium polyribosylribitol phosphate, lipopolysaccharide and various lipids and proteins, is mixed with HP20 (highly porous styrene and divinylbenzene copolymer) resin, sodium citrate, and deoxycholate, under suitable conditions. The lipopolysaccharide binds to the resin which is thereafter removed. The filtrate is diafiltered, the retentate recovered, and polyribosylribitol phosphate precipitated from solution with alcohol. The precipitate is centrifuged, the resulting pellet triturated with ethanol and acetone, and resulting solution vacuum dried. In the process, the detergent breaks the association of the aggregated lipopolysaccharide. The chelating agent ties up the divalent calcium ions so the vesicular structure of the lipopolysaccharide cannot be maintained, and also serves as a buffer to maintain the pH above about 8, mainly to prevent detergent gelation. The lipopolysaccharide is then able to bind hydrophobically to the resin. The PRP, which does not bind to the resin, remains free in solution and can be recovered in the filtrate. The membrane diafiltrations which follow remove the detergent and chelating agent from the solution, and the PRP is then precipitated and dried in a typical manner.

Polysaccharides or polysaccharide solutions from which endotoxin is removed in accordance with the present invention may contain any bacterial polysaccharides with acid groups, but are not intended to be limited to any particular types. Examples of such bacterial polysaccharides include Haemophilus influenzae (H. flu) type b polysaccharide; Neisseria meningitidis (meningococcal) groups A, B, C, X, Y, W135 and 29E polysaccharides; and Escherichia coli K1, K12, K13, K92 and K100 polysaccharides. Particularly preferred polysaccharides, however, are those capsular polysaccharides selected from the group consisting of H. flu b polysaccharide, such as described in Rosenberg et al., J. Biol. Chem., 236, pp. 2845-2849 (1961) and Zamenhof et al., J. Biol. Chem., 203, pp. 695-704 (1953).

H. influenzae type b polyribosylribitol phosphate, shown below,

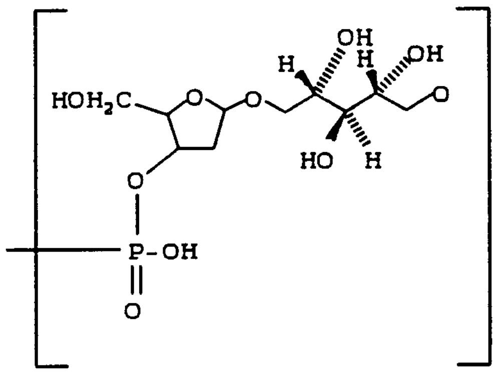

may be prepared for use in protein-polysaccharide conjugates such as those described in Marburg et al., United States Patent No. 4,695,624.

In another embodiment of the invention, a powder derived from H. influenzae fermentation broth containing polyribosylribitol phosphate, lipopolysaccharides, and various lipids and proteins is dissolved in a detergent/chelating agent mixture under basic pH. The resin beads are added to and mixed with the PRP solution in an orbital shaker for several hours below room temperature. The beads are then removed from solution, and the filtrate is diafiltered to remove the detergent and chelating agent. Retentate is recovered and calcium chloride added. The PRP is precipitated from solution with ethanol. The precipitate is centrifuged and the pellet is triturated with ethanol and acetone. The resulting product is vacuum dried. The process using resin beads results in low levels of contaminating endotoxin without significant loss of PRP. Endotoxin reduction resulting from the process of the invention is typically 100-21,000 fold between starting and final powder. PRP yield is typically at least 75% and sometimes more than 90% of the starting material.

The process of the invention for removing endotoxin and preparing endotoxin-free polysaccharide is particularly advantageous when preparing polysaccharides for conjugation with proteins. Our studies have shown that conjugation efficiency is three times better with polysaccharide prepared using the process of the present invention than with polysaccharide prepared according to Marburg et al., U.S. Patent No. 4,695,624. During preparation, the resin hydrophobically attaches to the lipid site of the lipopolysaccharide endotoxin to be removed, but does not attach in substantial quantities to other lipids that may be present. These other lipids remain in the polysaccharide solution and enhance the polysaccharide protein conjugation efficiency.

In another embodiment of the invention, lipopolysaccharide is removed from a solution containing polysaccharide derived from H. influenzae fermentation broth by mixing it with resin, e.g. HP20 (highly porous styrene and divinylbenzene copolymer) resin, a chelating agent, e.g. sodium citrate, and a detergent, e.g. sodium deoxycholate, under suitable conditions, and removing the resin by filtration. The filtrate is diafiltered, the retentate recovered, and PRP precipitated from solution with ethanol. The precipitate is centrifuged, the resulting pellet triturated with ethanol and acetone, and resulting solution vacuum dried. In the process, the detergent breaks the association of the aggregated lipopolysaccharide. The chelating agent ties up the divalent calcium ions so the vesicular structure of the lipopolysaccharide cannot be maintained, and also serves as a buffer to maintain the pH above about 8, mainly to prevent detergent gelation. The presence of both the detergent and chelating agent is essential for obtaining the superior results achievable with this process. The lipopolysaccharide is then able to bind hydrophobically to the resin. The PRP, which does not bind to the resin because it is too large to enter the pores and is hydrophilic, remains free in solution and can be recovered in the filtrate. The membrane diafiltrations which follow remove the detergent and chelating agent from the solution, and the PRP is then precipitated and dried.

The resin is mixed with a chelating agent such as sodium citrate, and a detergent such as deoxycholate. The detergent breaks the aggregated lipopolysaccharide. The chelating agent acts on divalent calcium ions present in the solution and serves as a buffer for maintaining basic pH.

A production fermenter containing complete Haemophilus medium with an antifoaming agent is inoculated with the seed culture. The fermenter is maintained at 37 ± 3 °C for a minimum of twelve hours with moderate aeration and agitation. The H. influenzae type b culture is inactivated after the fermentation is

completed by addition of thimerosal under agitation. Cells are removed by centrifugation or filtration and discarded. The culture supernatant is concentrated by ultrafiltration and additional impurities are removed by alcohol fractionation.

The high molecular weight species precipitate is dissolved in calcium chloride solution and a minimum of one additional alcohol fractionation is completed as described above to remove additional impurities. The second alcohol precipitate is collected by centrifugation and a dry powder is obtained by resuspending the precipitate in absolute ethanol followed by filtration, acetone wash and drying.

The powder is dissolved in sodium acetate solution and extracted several times with phenol to remove impurities. The aqueous solution containing polysaccharide is diafiltered with water to remove phenol. Calcium chloride solution is added to the solution and high molecular weight species are precipitated with alcohol and collected by centrifugation. The post phenol powder is resolubilized in calcium chloride solution and is then subjected to selective alcohol fractionation.

Incremental alcohol addition is an effective process for reducing the level of endotoxin to the point where it meets product specification, while minimizing the loss of polysaccharide from solution. By changing the alcohol concentration, different molecular weight species become insoluble and precipitate out of solution. Increasing alcohol concentration precipitates species of decreasing molecular weight. When the cloud point is reached, lipopolysaccharide and polyribosylribitol phosphate begin to precipitate. Lipopolysaccharide is precipitated along with some polysaccharide, leaving polysaccharide in solution essentially unaccompanied by lipopolysaccharide. The low ethanol precipitate, which contains large quantities of lipopolysaccharides, is removed by centrifugation and discarded. Additional ethanol is added and the polysaccharide precipitate is collected by centrifugation. By following the procedure of the present invention, most lipopolysaccharide can be removed from solution before experiencing intolerable losses of polysaccharide. The resulting product is then suitable for efficient preparation of protein-polysaccharide conjugates.

Protein-polysaccharide conjugates useful for vaccination of patients against infections such as those caused by H. influenzae type b bacterium may be prepared using the process of the invention.

Endotoxin reduction resulting from the process of the invention is typically 30-100 fold between starting and final powder. Polyribosylribitol phosphate yield is typically at least 35% of the level in the starting material.

The limulus ameobocyte lysate (LAL) test, described in "Guideline on validation of the LAL test as an end-product endotoxin test for human and animal parenteral drugs, biological products, and medical devices." U.S. Department of Health and Human Services, December 1987, is used to determine endotoxin levels.

EXAMPLE 1

Endotoxin Removal Using Alcohol Fractionation and Resin Elimination

A schematic representation of the process followed in this example is shown below.

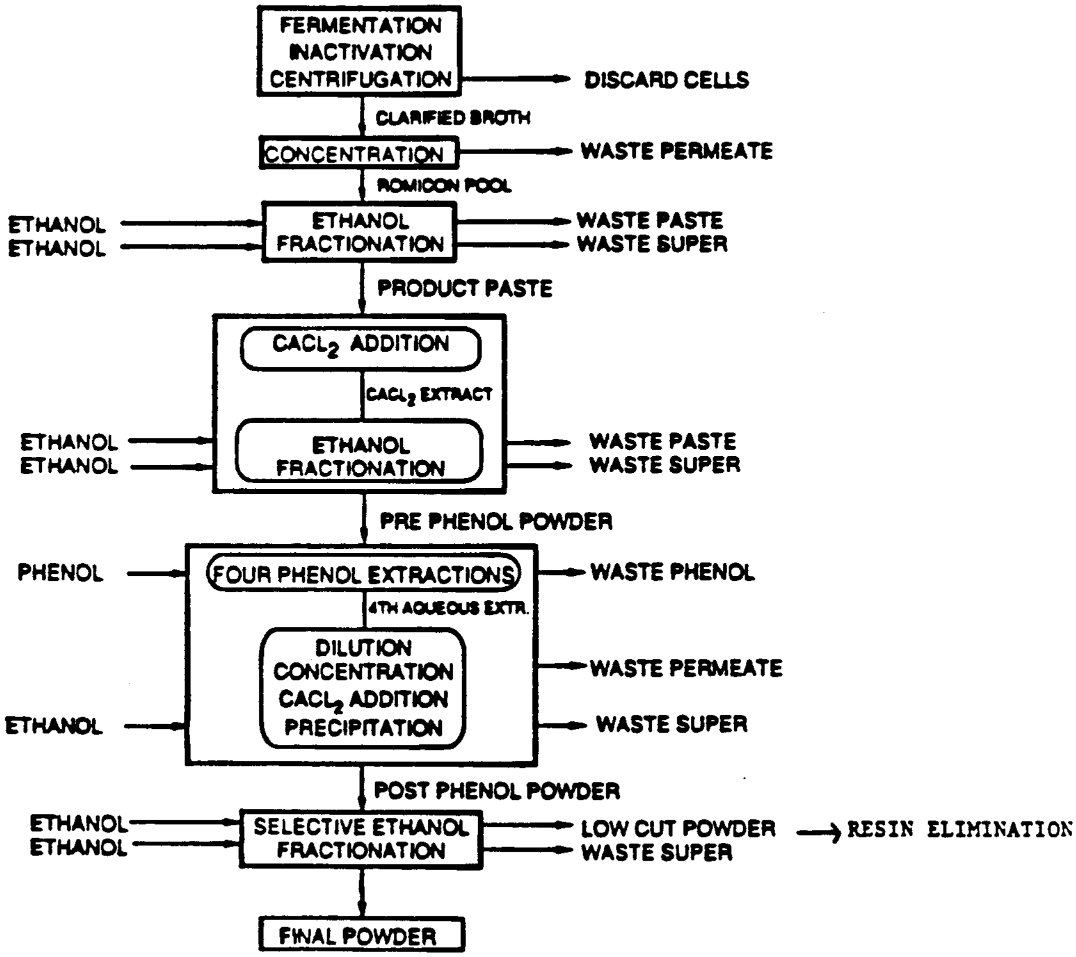


In the selective ethanol fractionation step, the lipopolysaccharide was precipitated as alcohol concentration increased, along with some polysaccharide, leaving polysaccharide in solution with reduced lipopolysaccharide. Precipitate containing lipopolysaccharide along with polysaccharide is known as the "low-cut" or "pre-cut". The pre-cut material is subjected to further endotoxin removal using resin, detergent, and chelating agent (as described later).

Incremental addition of alcohol is an effective process for reducing the level of endotoxin to the point where it meets product specification, while minimizing the loss of polysaccharide from solution. By changing the alcohol concentration, different molecular weight species become insoluble and precipitate out of solution. Increasing alcohol concentration precipitates species of decreasing molecular weight.

Thus, the solution from which endotoxin is to be removed is cooled and a salt such as $CaCl_2$ or NaCl is added. Chilled alcohol, such as SDA3A, is added to achieve a concentration slightly below (about 0.5-1.0%) less than the cloud point (see Graph 2). Sequential addition thereafter of about 0.2% alcohol at a time is performed until a two-fold increase in turbidity occurs, at which point the cloud point has been reached.

Products obtained from Tests a, c, d, and e in Table 1 show dramatic reductions of endotoxin level following the process of the invention. Test e, which had an unacceptably high level of endotoxin, was treated a second time by selective ethanol fractionation, the results of which are shown in test f.

TABLE 1

| Endotoxin Reduction Using Selective Alcohol Fractionation | | | | | | |
|---|---|---|---|---|---|---|
| | Test (EU/µg ) | | | | | |
| | a | b | c | d | e | f |
| Pre-phenol Powder | 750 | 650 | 530 | 600 | 780 | - |
| Post-phenol Powder | 45 | 140 | 60 | 60 | 135 | - |
| Low cut Powder | 30 | 600 | 340 | 30 | 300 | - |
| Powder After Selective Alcohol Fractionation | 1.5 | 0.9 | 1.4 | 0.4 | 2.8 | 0.09 |

To accomplish the selective alcohol fractionation, the post-phenol powder was solubilized at 2.5 g/L in a 0.05 M $CaCl_2$ solution to provide a divalent counter ion for both endotoxin and PRP. Alcohol was then added to 26% (v/v). After the temperature equilibrated to a constant value in the 2 to 4°C range, alcohol was added incrementally until the PRP begins to precipitate (cloud point), causing turbidity as monitored by a turbidity probe.

Graph 1 is a plot of % alcohol at the cloud point versus the temperature of a PRP powder solution. The % alcohol needed to reach the cloud point at 6°C was 27.4% but for the 4°C only 26.7% was required. This seemingly small increase corresponded to 700 ml for a 100 L scale run. The final powder yield decreased as the difference between Low Cut Alcohol percent and Cloud Point percent increased. Graph 2 shows that an increase in alcohol content of 1% from the cloud point alcohol concentration removed 50% of the PRP. Endotoxin reduction, as measured by LAL, was about ten fold. Therefore, alcohol addition of 1% was not sufficient to reduce the endotoxin to a level of 3 EU/µg when the starting LAL was greater than 30 EU/µg.

After the low cut alcohol was added, the solution was immediately centrifuged to remove low cut precipitate. Additional alcohol was added to the supernatant to 38% (v/v). The desired precipitate was collected via settling and/or centrifugation and dried to the final powder. Typical recoveries for this step at 1.2-2.0% above the cloud point were 30-40% of the post-phenol powder or 13-18% of the amount from the fermentor.

The selective alcohol fractionation procedure can be repeated if the final powder does not meet the pyrogen specification. For reprocessing, the alcohol concentration was increased 0.2% above the low cut alcohol percentage. The yield was 78% and the endotoxin level was reduced from 2.8 to 0.09EU/µg.

Graph 1

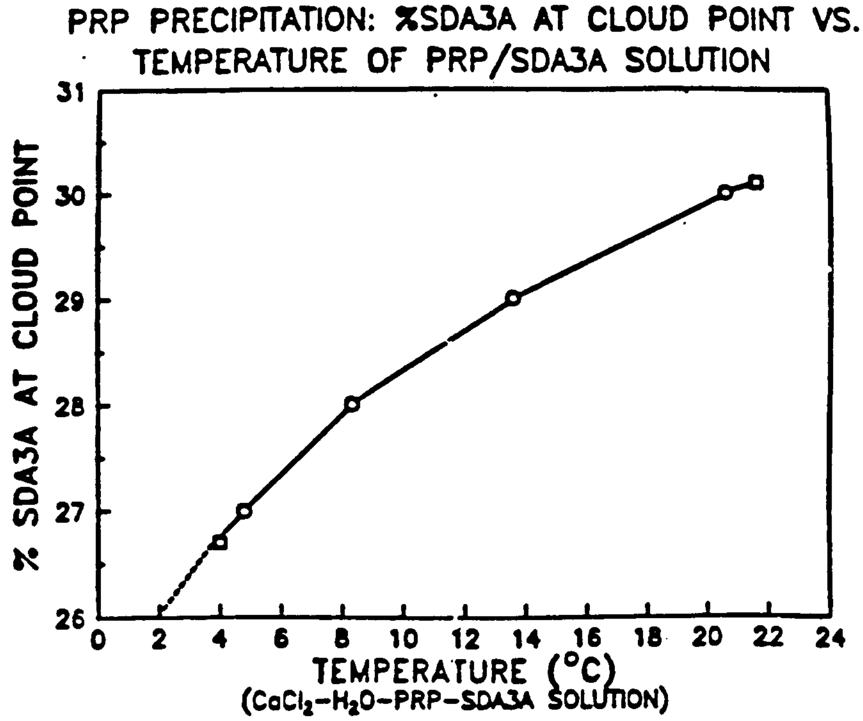


Graph 2

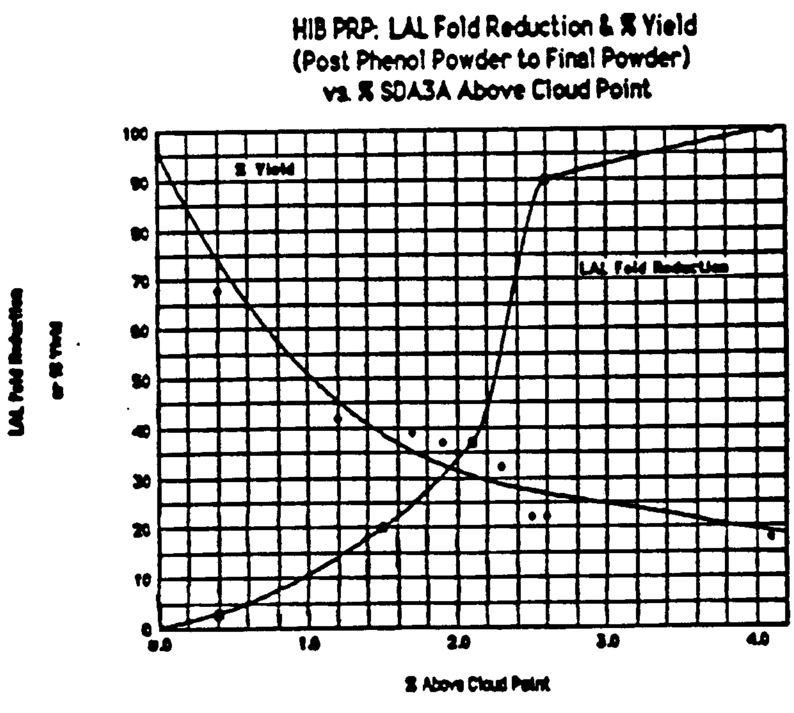


Endotoxin Removal Using Resin, Detergent and Chelating Agent

The pre-cut or low-cut material obtained after performing the selective ethanol fractionation step, containing precipitated lipopolysaccharide and polyribosylribitol phosphate, was further treated by mixing with HP20 resin, deoxylcholate and sodium citrate. This treatment removes substantial quantities of

lipopolysaccharide without significantly affecting the level of desirable polyribosylribitol phosphate contained in the low cut material. The filtrate is diafiltered with a hollow fiber membrane, the retentate recovered, and polyribosylribtol phosphate precipitated from solution with ethanol. The precipitate is centrifuged and resulting pellet triturated with ethanol and acetone. The resulting solution is then vaccum dried.

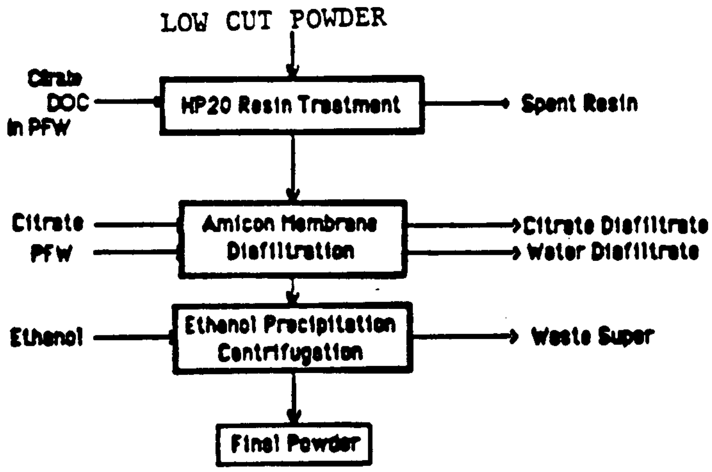


0.5% sodium deoxycholate and 3% sodium citrate were mixed with the lipopolysaccharide-polysaccharide mixture at pH 8-9. HP20® resin was added at 30 grams resin per gram polysaccharide (the resin was washed prior to use with pyrogen free water). The loose beads were mixed with the solution on an orbital shaker for 3 hours at 4°C. After mixing, the beads are removed from the solution in a stainless steel filter funnel. Filtrate is then diafiltered in an Amicon H1P30-20 hollow fiber cartridge ($0.06M^2$ surface area) vs. 5 vol. of 1.5% citrate followed by 10 vol. of pyrogen free water, maintaining an estimated polysaccharide concentration of ≦ 2.5 mg/ml, to remove detergent and chelating agent. The retentate is recovered and 2M calcium chloride is added to achieve a final calcium chloride concentration of 0.05 M. Polysaccharide is precipitated from solution with excess 95% ethanol. The precipitate is centrifuged at 13,000 x g for 30 minutes, the pellet triturated with absolute ethanol and acetone, and then vacuum dried. The final powder is transferred to a sample container and frozen at -70°C.

Material treated with resin showed the following reductions of endotoxin levels and polysaccharide levels:

| LAL test value EU/µg | A | B | C |
|---|---|---|---|
| initial -<br>final powder - | 100<br>0.06 | 100<br>0.03 | 100<br>0.06 |
| Polysaccharide level (%) | | | |
| initial -<br>final powder - | 100<br>90 | 100<br>100 | 100<br>92 |

<u>EXAMPLES 2, 3, 4, 5, 6, 7 and 8</u>

Following the procedure for endotoxin removal described in Example 1, maintaining a concentration of sodium deoxycholate of 0.5%, and beginning with powder having LAL of 60 EU/µg, we obtained considerable reduction in LPS with these varying amounts of sodium citrate:

| | % Sodium Citrate | LAL (EU/µg ) | |
|---|---|---|---|
| | | Start | Finish |
| Example 2 | 2 | 60 | 30 |
| Example 3 | 3 | 60 | 6 |
| Example 4 | 4 | 60 | 0.6 |
| Example 5 | 5 | 60 | 0.6 |
| Example 6 | 6 | 60 | 0.15 |
| Example 7 | 7 | 60 | 0.6 |
| Example 8 | 8 | 60 | 0.6 |

EXAMPLES 9, 10 and 11

Following the procedure for endotoxin removal described in Example 1, maintaining a concentration of sodium citrate of 6%, and beginning with powder having LPS of 60 EU/µg, we obtained considerable reduction in LPS with these varying amounts of deoxycholate:

| | %Sodium Deoxycholate | LAL (EU/µg ) | |
|---|---|---|---|
| | | Start | Finish |
| Example 9 | 0.25 | 60 | 15 |
| Example 6 | 0.5 | 60 | 0.15 |
| Example 10 | 0.75 | 60 | 0.006 |
| Example 11 | 1.0 | 60 | 0.6 |

EXAMPLES 12, 13, 14, 15 AND 16

Following the general procedures in Example 1, these examples include description or process variations within the invention.

Process Variation

| | |
|---|---|
| Example 12 | After treatment in accordance with Example 1, the procedure is repeated. |
| Example 13 | After treatment according to Example 3, filtrate is diafiltered with 3% sodium citrate, sodium deoxycholate is added, and the solution treated for three additional hours with the original HP-20 resin. |
| Example 14 | After three hours of HP-20 treatment according to Example 3, an equal volume of 3% citrate with 0.5% sodium deoxycholate was added to the mixture and resin treatment continued for another three hours. |
| Example 15 | After three hours according to Example 3, sodium citrate with 0.5% sodium deoxycholate powder were added and the resin treatment continued for another three hours. |
| Example 16 a,b | (a) Six percent sodium citrate with 0.5% sodium deoxycholate or<br>(b) 6% sodium citrate with 1% sodium deoxycholate was used for three hours. |

EXAMPLE 17

All of the process steps of Example 1 are used, except that the resin is packed in a column, rather than mixed in batch with PRP. Thus, the PRP is dissolved in a solution of sodium citrate and sodium deoxycholate, and the resulting solution is passed through the column. The resulting product is similar to that obtained in Example 1.

EXAMPLE 18

All of the process steps of Example 1 are repeated, except that the resin is packed in a cartridge, rather than mixed in batch with PRP. Thus, the PRP is dissolved in a solution of sodium citrate and sodium deoxycholate, and the resulting solution is passed through the cartridge. The resulting product is similar to that obtained in Example 1.

EXAMPLE 19

## Preparation of *H. Influenzae* Type b Capsular Polysaccharide (PRP)

A schematic representation of the process is shown below:

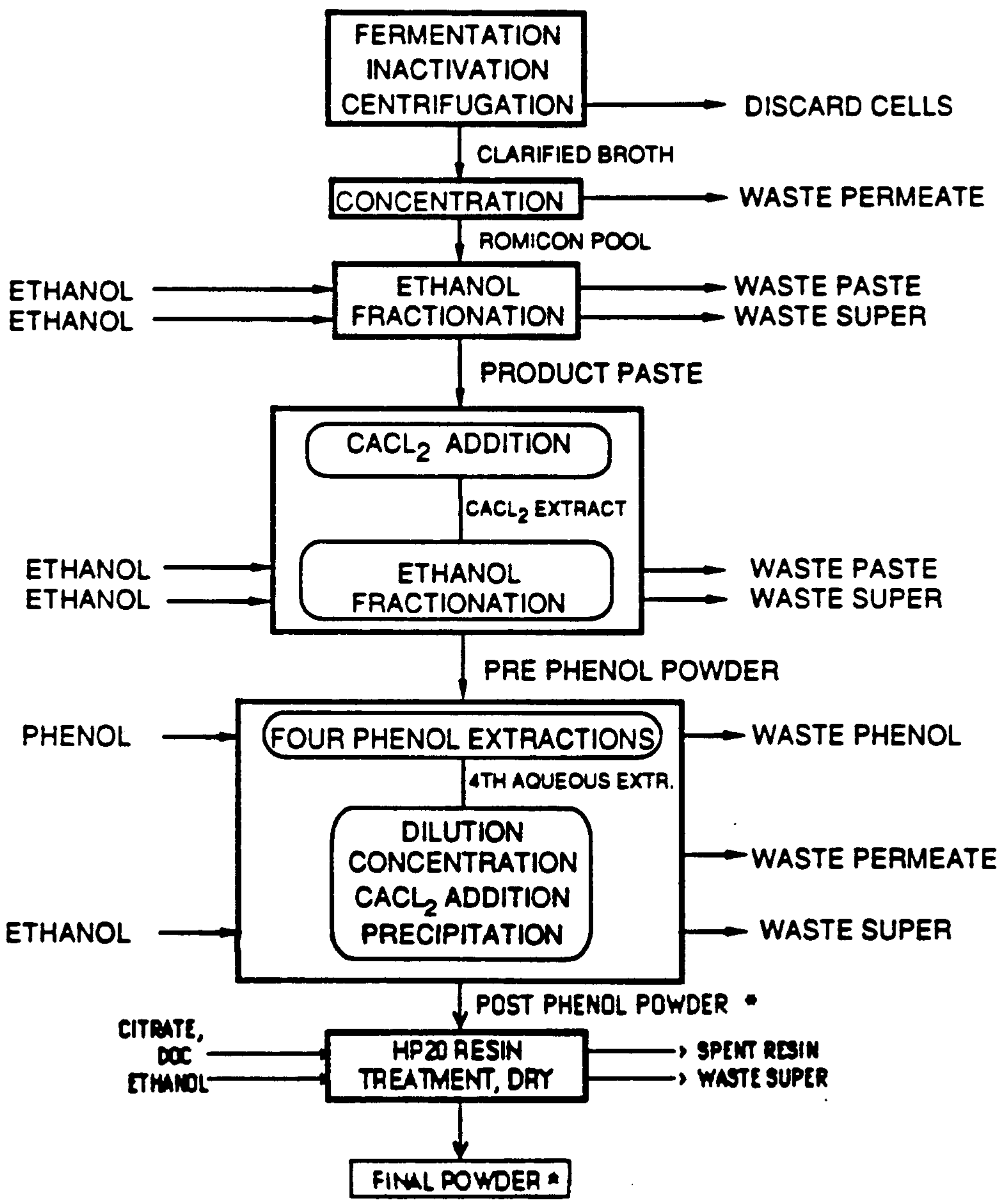

Fermentation

A Stage: Inoculum and Seed Development

A lyophilized tube of Haemophilus influenzae type b, (cultured from Ross 768, received from State University of New York) was suspended in 1 ml of sterile Haemophilus inoculum medium (see below) and this suspension was spread on nineteen Chocolate Agar Plates (BBL). After 20 hours incubation at 37°C in a candle jar, the growth on each plate was resuspended in 1-2 ml Haemophilus inoculum medium and pooled.

| Haemophilus Inoculum Medium* | |
|---|---|
| Soy Peptone | 10 gm/liter |
| NaCl | 5 gm/liter |
| $NaH_2PO_4$ | 3.1 gm/liter |
| $Na_2HPO_4$ | 13.7 gm/liter |
| $K_2HPO_4$ | 2.5 gm/liter |
| Distilled Water | To Volume |

*The pH of the solution is adjusted to a target value of 7.2±0.1 (a typical value was pH 7.23) and the solution was sterilized by autoclaving at 121°C for 25 minutes.

B Stage: 2-Liter Non-baffled Erlenmeyer Flasks

One-third portions of the resuspended bacteria from "A Stage" (above) were used to inoculate three two-liter flasks, each containing about 1.0 liter of complete Haemophilus seed and production medium (see below). The flasks were then incubated at 37°C on a rotary shaker of 200 rpm for about 5 hours. A typical $OD_{660}$ value at the end of the incubation period was 0.37.

| Complete Haemophilus Seed & Production Medium | |
|---|---|
| $NaH_2PO_4$ | 3.1 g/l |
| $Na_2HPO_4$ | 13.7 g/l |
| Soy Peptone | 10.0 g/l |
| Yeast extract diafiltrate (1) | 10.0 ml/l |
| $K_2HPO_4$ | 2.5 g/l |
| NaCl | 5.0 g/l |
| Glucose (2) | 5.0 g/l |
| Nicotinamide adenine dinucleotide (NAD) (3) | 2.0 mg/l |
| Hemin (4) | 5.0 mg/l |

The salts and soy peptone were dissolved in small volumes of hot, pyrogen-free water and brought to correct final volume with additional hot, pyrogen-free water. The fermenters or flasks were then sterilized for about 25 minutes at 121°C and after cooling, yeast extract diafiltrate (1), glucose (2), NAD (3), and hemin (4) were added aseptically to the flasks or fermenters prior to inoculation.

(1) Yeast extract diafiltrate: 100 g brewers' yeast extract (Amber) was dissolved in 1 liter distilled water and ultrafiltered in an Amicon DC-30 hollow fiber with H10X50 cartridges to remove molecules with m.w. 50,000. The filtrate was collected and passed through a 0.22 M membrane as a sterile product.

(2) Glucose was prepared as a sterile 25% solution in glass-distilled water.

(3) A stock solution of NAD containing 20 mg/ml was sterilized by filtration through a Millipore filter (0.22 M) and added aseptically just prior to inoculation.

(4) A stock solution of Hemin 3X was prepared by dissolving 200 mg in 10 ml of 0.1 M NaOH and the volume adjusted with distilled, sterilized water to 100 ml. The solution was sterilized for 20 minutes at 121°C and added aseptically to the final medium prior to inoculation.

C Stage: 70-Liter Seed Fermenter

Three liters of the product of "B Stage" was used to inoculate a 70-liter fermenter containing 41.4 liters of complete haemophilus seed and production medium (prepared as described above) and 17 ml UCON B625 antifoam. The pH started at 7.4.

The fermentation was maintained at 37°C with 100 rpm agitation and monitored by optical density (O.D.) and pH determination until a typical O.D. of 0.39 was reached (after about 5.5 hours).

D Stage: 800-Liter Production Fermenter

Approximately 40 liters of the product of "C Stage" was used to inoculate an 800-liter fermenter containing 570 liters of production medium (prepared as described above), scaled to the necessary volume and 72 ml of UCON LB625 antifoam.

The fermentation was maintained at 37°C with 100 rpm of agitation, with the O.D. and pH levels being checked about every two hours until the O.D. was similar for a two-hour period, at which time the fermentation was terminated (a typical final O.D. was 0.54 after 12 hours).

Harvest and Inactivation

Approximately 600 liters of the batch was inactivated by harvesting into a "kill tank" containing 12 liters of 1% thimerosal.

Clarification

After 8 hours inactivation at 4°C, the batch was centrifuged in 4-in. (101.6 mm) bowl Sharples centrifuges at a flow rate adjusted to maintain product clarity (variable between 1.3 and 3.0. liters/min). The supernatant obtained after centrifugation (15,000 rpm) was used for product recovery.

Isolation and Concentration by Ultrafiltration

The supernatant fluid from two production fermentations was pooled and concentrated at 2°-8°C in a Romicon ultrafiltration unit with ten (50,000 Daltons cut-off) hollow fiber cartridges (275 $ft^2$ [25.55 $m^2$] membrane area) such that after approximately 4.5 hours, 1200 liters had been concentrated to 32.5 liters. The filtrate was discarded.

48% and 61% Ethanol Precipitation

To the 32.5 liters of Romicon retentate, 30 liters of 95% ethanol was added dropwise over 1 hour with stirring at 4°C to a final concentration of 48% ethanol by volume. The mixture was stirred two additional hours at 4°C to ensure complete precipitation, and the supernatant fluid was collected through a single 4-inch (101.6 mm) Sharples centrifuge at 15,000 rpm (flow rate = 0.27 liters/min). The insoluble pellet was discarded and the clarified fluid was brought to 61% ethanol with the addition of 20.8 liters of 95% ethanol over a one hour period. The mixture was stirred for three additional hours to insure complete precipitation.

Recovery of the Second Pellet

The resulting 48% ethanol soluble-61% ethanol insoluble precipitate was collected by centrifugation in the 4-inch (101.6 mm) Sharples centrifuge at 15,000 rpm (flow rate = 0.62 liters/min.) and the 61% ethanol supernatant fluid was discarded. The crude product yield was 0.377 kg of wet paste.

Calcium Chloride Extraction

The 377 grams of 61% ethanol insoluble material, was mixed in a Daymax dispersion vessel at 2°-8°C with 6.5 liters of cold, glass-distilled water. To this mixture, 6.5 liters of cold 2M $CaCl_2$"$H_2O$ was added, and the mixture (final concentration = 1.0 M $CaCl_2$) was extracted at 4°C for 15 minutes. The vessel was then rinsed out with 2 liters of 1 M $CaCl_2$"$H_2O$, resulting in 15 liters final volume.

23% Ethanol Precipitation

The 15 liters of $CaCl_2$ extract from above was brought to 23% ethanol by adding 4.48 liters of 95% ethanol dropwise, with stirring, at 4°C over 30 minutes. After additional stirring for 17 hours, the mixture was centrifuged through a K2 Ultracentrifuge at 25,000 rpm (flow rate - 165 ml/min) for 6.5 hours at 4°C. The supernatant fluid was decanted through cheese cloth to remove lipid-like floating material and the insoluble pellet was discarded.

37% Ethanol Precipitation and Collection of Crude Product Paste

The 23% ethanol-soluble supernatant fluid was brought to 37% ethanol by the addition of 4.33 liters of 95% ethanol, dropwise with stirring, over a 30 minute period. The mixture was then allowed to stand with agitation for one hour, then without agitation for 14 hours, to ensure complete precipitation. The resulting mixture was then centrifuged in a 4-inch (101.6 mm) Sharples unit at 15,000 rpm (flow rate = 0.2 liters/min) to collect the pelleted crude polysaccharide (referred to hereinafter as pre-phenol powder).

Trituration

The pellet from the centrifugation was transferred to a 1-gallon (3.8 litre) Waring Blender containing 1 liter of absolute alcohol and blended for 30 seconds at the highest speed. Blending was continued at 30 seconds on and 30 seconds off until a hard, white powder resulted. The powder was collected on a Buchner funnel with a teflon filter disc and washed sequentially in situ with two 1-liter portions of absolute ethanol and two 2-liter portions of acetone. The material was then dried in vacuo, at 4°C, for 24 hours, resulting in 68 g (dry weight) of product.

Phenol Extraction

The 68 grams of dry material from the trituration step was resuspended in 12 liters of 0.488 M sodium acetate, pH 6.9, with the aid of a Daymax dispersion vessel. The sodium acetate solution was immediately extracted with 4.48 liters of a fresh aqueous phenol solution made as follows: 900 ml of 0.488 M sodium acetate, pH 6.9, was added to a five-pound bottle of phenol (Mallinckrodt crystalline) in a 20-liter pressure vessel and mixed until a complete solution was effected. Each phenol extract was centrifuged for 2 1/2 hours at 30,000 rpm and 4°C in the K2 Ultracentrifuge (Electronucleonics) in order to break the emulsion. The aqueous effluent was extracted three additional times with 3.2 fresh aqueous phenol solution in a similar manner. The phenol phases were discarded.

Diafiltration

The aqueous phase from the phenol extractions above (17.6 liters) was diluted with 300 liters of cold, glass-distilled water and diafiltered at 4°C on an Amicon DC-30 ultrafiltration apparatus using 3 H10P10 cartridges. The Amicon unit was rinsed and the rinse added to the retentate, such that the final volume was 17.5 liters. The ultrafiltrate was discarded.

67% Ethanol Precipitation

0.438 liters of 2.0 M $CaCl_2$ was added to the 17.5 liters of dialysate from the previous step (final $CaCl_2$ concentration was 0.05 M) and the solution was made 67% ethanol with dropwise addition over one hour of 35.88 liters of 95% ethanol to the rapidly-stirring solution. After 4 hours of agitation, then standing for 12 hours more at 4°C, the clear supernatant fluid was siphoned off and the precipitate was collected by centrifugation in the 4-inch (101.6 mm) Sharples centrifuge (15,000 rpm), at 4°C for 45 minutes. The resulting polysaccharide pellet was triturated in a 1-gallon (3.8 litre) Waring blender using the 30 seconds on 30 seconds off method with 2 liters of absolute ethanol, collected on a Buchner funnel fitted with a teflon filter disc, and washed in situ with four 1-liter portions of absolute ethanol followed by two 1-liter portions of acetone. The sample was then dried in a tared dish in vacuo at 4°C for 20 hours. The yield was 39 grams of dry powder (referred to hereinafter as post-phenol powder).

Endotoxin Removal

A schematic of the procedure is shown below.

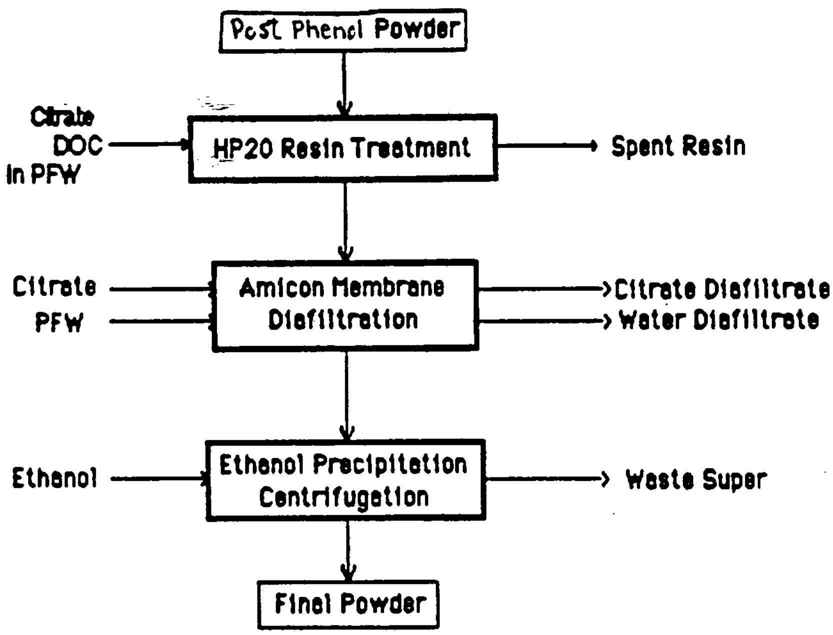


Endotoxin Removal

A schematic of the procedure is shown below. PRP post-phenol powder was dissolved, at 2 mg/ml, in a solution of 3% sodium citrate and 0.5% sodium deoxycholate at pH 8-9. HP20® resin was added at 30 grams resin per gram PRP (the resin was washed prior to use with the pyrogen free water). The loose beads were mixed with the PRP solution on an orbital shaker for 3 hours at 4°C. After mixing, the beads are removed from the solution in a stainless steel filter funnel. Filtrate is then diafiltered in an Amicon H1P30-20 hollow fiber cartridge ($0.06m^2$ surface area) vs. 5 vol. of 1.5% citrate followed by 10 vol. of pyrogen free water, maintaining an estimated PRP concentration of ≦ 2.5 mg/ml to remove detergent and chelating agent. The retentate is recovered and 2 M calcium chloride is added to achieve a final calcium chloride concentration of 0.05 M. PRP is precipitated from solution with excess 95% ethanol. The precipitate is centrifuged at 13,000 x g for 30 minutes, the pellet triturated with absolute ethanol and acetone, and then vacuum dried. The final powder is transferred to a sample container and frozen at -70°C.

Endotoxin level was reduced between 100 and 21,000 fold between starting and final powders. PRP yield is between 75 and > 90 % from starting to final powder.

900 mg of post-phenol powder was processed. Quantitative determination of endotoxin was done using the well-known limulus ameobocyte lysate (LAL) test. LAL was reduced after the various process steps as

shown below (1 EU/µg of polysaccharide = 100 ppm):

LAL test value (ppm)

initial - 18,000
after HP20® resin treatment - 12
after Hollow Fiber treatment - 12
final powder - 6

PRP loss caused by the process was not substantial. Amounts of PRP remaining at various stages of the process, represented by percentage of the initial amount, are shown below:

initial - 100%
after HP20® resin treatment - 93
after Hollow Fiber treatment - 90
final powder - 75

EXAMPLES 20, 21, 22, 23, 24 and 25

Following the procedure for endotoxin removal described in Example 19, maintaining a concentration of sodium deoxycholate of 0.5%, and beginning with powder having LPS of 260 EU/µg, we obtained the following reduction in LAL with these varying amounts of sodium citrate:

| | % Sodium Citrate | LPS EU/µg |
|---|---|---|
| Example 20 | 2 | 22 |
| Example 21 | 3 | 4.2 |
| Example 22 | 4 | 1.0 |
| Example 23 | 5 | 0.6 |
| Example 24 | 6 | 0.9 |
| Example 25 | 7 | 0.6 |

EXAMPLES 26, 27 AND 28

Following the procedure for endotoxin removal described in Example 19, maintaining a concentration of sodium citrate of 6%, and beginning with powder having LPS of 260 EU/µg, we obtained the following reduction in LAL with these varying amounts of deoxycholate:

| | % Sodium Deoxycholate | LPS EU/µg |
|---|---|---|
| Example 26 | 0.25 | 15 |
| Example 24 | 0.5 | 0.9 |
| Example 27 | 0.75 | 0.01 |
| Example 28 | 1.0 | 0.4 |

EXAMPLES 29, 30, 31, 32 AND 33

Following the general procedure in Example 19, these examples include description of process variations and the resulting LPS obtained from powder originally having 260 EU/µg.

Process Variation

| | | LPS Avg. EU/μg |
|---|---|---|
| Example 29 | After treatment in accordance with Example 3, the procedure is repeated. | 0.4 |

| | | |
|---|---|---|
| Example 30 | After treatment according to Example 3, filtrate is diafiltered with 3% sodium citrate, sodium deoxycholate is added, and the solution treated for three additional hours with the original HP-20 resin. | 0.5 |
| Example 31 | After three hours of HP-20 treatment according to Example 3, an equal volume of 3% citrate with 0.5% sodium deoxycholate was added to the mixture and resin treatment continued for another three hours. | 0.4 |
| Example 32 | After three hours according to Example 3, sodium citrate and sodium deoxycholate powder were added and the resin treatment continued for another three hours. | 0.2 |
| Example 33 a,b | (a) Six percent sodium citrate with 0.5% sodium deoxycholate or (b)6% sodium citrate with 1% sodium deoxycholate was used for three hours. | (a) 0.3<br>(b) 0.1 |

EXAMPLE 34

All of the process steps of Example 1 are used, except that the resin is packed in a column, rather than mixed in batch with PRP Thus, the PRP is dissolved in a solution of sodium citrate and sodium deoxycholate, and the resulting solution is passed through the column. The resulting product is similar to that obtained in Example 19.

EXAMPLE 35

All of the process steps of Example 1 are repeated, except that the resin is packed in a cartridge, rather than mixed in batch with PRP. Thus, the PRP is dissolved in a solution of sodium citrate and sodium deoxycholate, and the resulting solution is circulated through the cartridge. The resulting product is similar to that obtained in Example 19.

EXAMPLE 36

In this example, all of the process steps of Example 19 are followed. In accordance with this procedure, 1 gram of PRP is processed. Results are set forth below:

LAL test value (ppm)

initial - 11,000
after HP20 resin treatment - 15
after Hollow Fiber treatment - 11.2
final powder - 6

PRP loss caused by the process was not substantial. Amounts of PRP remaining at various stages of the process, represented by percentage of the initial amount, are shown below:

initial - 100%
after HP20® resin treatment - 96
after Hollow Fiber treatment - 93
final powder - 91

The material obtained by this process conformed to all current chemical specifications.

EXAMPLE 37

In this example, the method of Example 19 is followed except that 6% sodium citrate and 0.75% sodium deoxycholate are used instead of 3% sodium citrate and 0.5% sodium deoxycholate. The method was applied to four different lots of post-phenol powder material, (a, b, c and d). LAL test value (EU/µg ) reductions achieved are shown below:

| LAL test value (EU/µg ) | | |
|---|---|---|
| | Initial | Final |
| a. | 22 | 0.0012 |
| b. | 126 | 0.006 |
| c. | 190 | 0.009 |
| d. | 270 | 0.012 |

EXAMPLE 38

## H. Influenzae Polysaccharide Isolation Process With Selective Ethanol Fractionation

A schematic representation of the process followed in this example is shown below.

*H. INFLUENZAE* **POLYSACCHARIDE ISOLATION PROCESS WITH SELECTIVE ETHANOL FRACTIONATION**

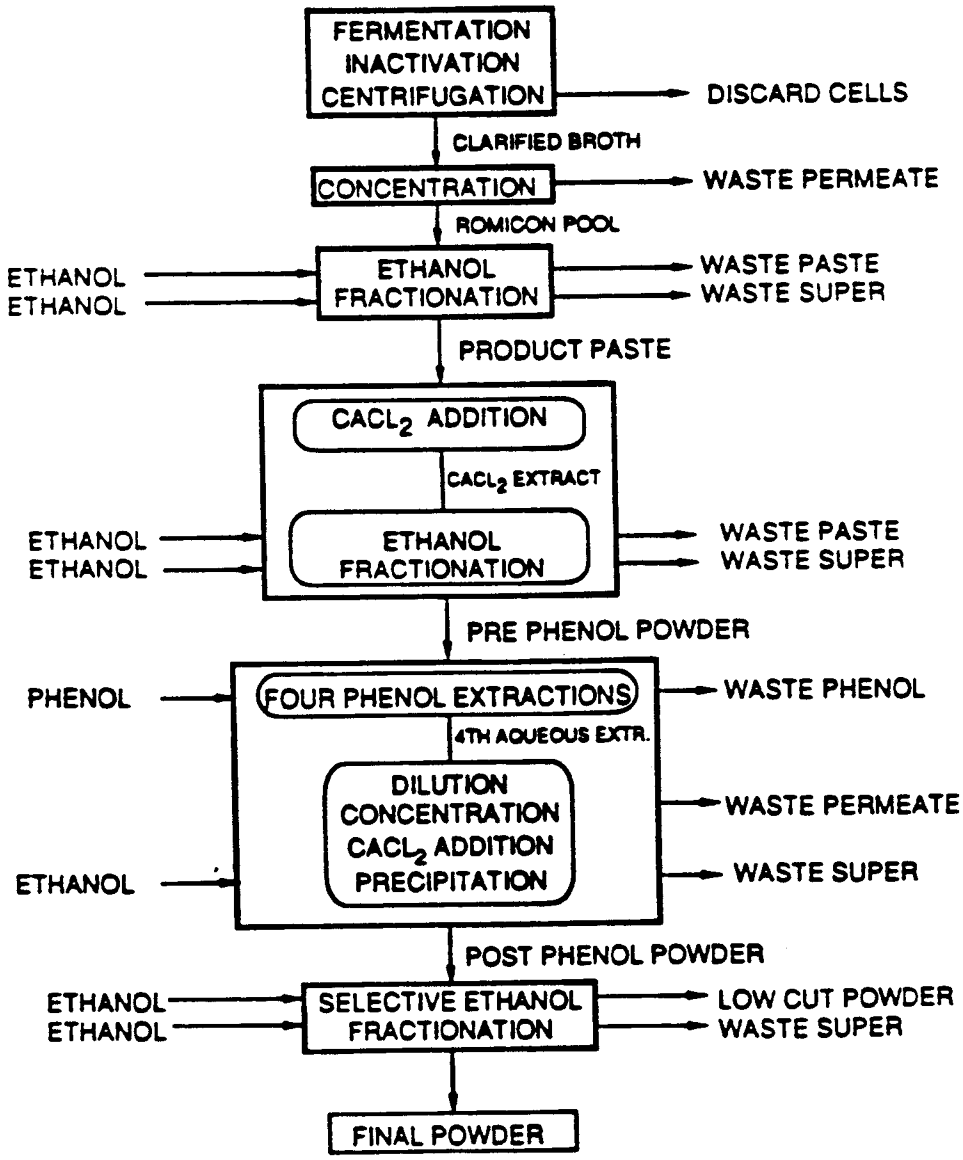


H. influenzae type b was grown in an 800 L fermentor (640 L working volume). A sample for culture purity was obtained and the culture transferred to a kill tank where it was treated with Thimerosal. At the completion of the kill cycle (10 hours at 37 °C), the temperature was reduced and the broth held until released by the culture viability test (30 hours). The inactivated whole broth was then transferred out of the containment area, the cells and other debris removed by Sharples centrifugation, and the clarified broth stored at 2-8 °C. Since the PRP was released into the culture media, the collected cells were discarded after weighing. The dilute cell-free broth is concentrated and a first ethanol fractionation is performed to

remove contaminating protein, nucleic acid and endotoxin. A second ethanol fractionation is then performed to further purify the concentrated broth, followed by a series of phenol extractions to remove residual protein, endotoxin and pigments. These fractionations and extractions result in material which contains undesirable amounts of endotoxin.

In the selective ethanol fractionation step, the lipopolysaccharide was precipitated as alcohol concentration increased, along with some polysaccharide, leaving polysaccharide in solution which was lower in lipopolysaccharide level. Preciptate containing substantial quantities of lipopolysaccharide with polysaccharide is known as the "low-cut".

Thus, the solution from which endotoxin was to be removed was cooled and a salt such as $CaCl_2$ or NaCl was added. Chilled denatured alcohol was added to achieve a concentration slightly below (about 0.5-1.0% below) the cloud point (see Graph 1). Sequential addition thereafter of about 0.2% alcohol at a time was performed until a two-fold increase in turbidity occurred, at which point the cloud point was reached.

Products obtained from Tests a, c, d, and e in Table 1 show dramatic reductions of endotoxin levels following the process of the invention. Reduction of endotoxin level is measured by measuring limulus ameobocyte lysate (LAL) test values. The test is described in "Guideline on validation of the LAL test as an end product endotoxin test for human and animal parenteral drugs, biological products, and medicinal devices". U.S. Department of Health and Human Services, December 1987. Product from Test e, which had an unacceptably high level of endotoxin, was treated a second time by selective ethanol fractionation, the results of which are shown in the product from Test f.

To accomplish the selective ethanol fractionation, the post-phenol powder was solubilized at 2.5 g/L in a 0.05 M $CaCl_2$ solution to provide a divalent counter ion for both endotoxin and PRP. Alcohol was then added to achieve a level of 26% (v/v). After the temperature equilibrated to a constant value in the 2 to 4°C range, alcohol was added incrementally until the PRP began to precipitate (cloud point), causing turbidity as monitored by a turbidity probe.

Graph 1 is a plot of % alcohol at the cloud point versus the temperature of a PRP powder solution. The % alcohol needed to reach the cloud point at 6°C was 27.4% but for the 4°C only 26.7% was required. This seemingly small increase corresponded to 700 ml for a 100 L scale run. Historical data was used to decide how much additional alcohol should be added after the cloud point was reached in order to reduce the lipopolysaccharide to meet the specification. The final powder yield decreased as the difference between Low Cut Alcohol percent and Cloud Point percent increased. Graph 2 shows that an increase in alcohol content of 1% from the cloud point alcohol concentration removed 50% of the PRP. Endotoxin reduction, as measured by LAL, was about ten fold. Therefore, alcohol addition of 1% was not sufficient to reduce the endotoxin to a level of 3 EU/$\mu$g when the starting LAL was greater than 30 EU/$\mu$g.

After the low cut alcohol was added, the solution was immediately centrifuged to remove low cut. Additional alcohol was added to the supernatant to 38% (v/v). The desired precipitate was collected via settling and/or centrifugation and dried to the final powder. Typical recoveries for this step using 1.2 to 2% above cloud point were 30-40% of the post-phenol powder or 13-18% of the amount from the fermentor.

The selective alcohol fractionation procedure can be repeated if the final powder does not meet the pyrogen specification. For reprocessing the product from test e, the alcohol concentration was increased 0.2% above the low cut alcohol percentage. The yield was 78% and the endotoxin level was reduced from 2.8 to 0.09EU/$\mu$g.

The polysaccharide product resulting from the endotoxin removal procedure of the invention is especially useful where endotoxin-free polysaccharide polyribosylribitol phosphate is desirable. It readily conjugates to proteins, e.g. immunogenic proteins, such as in the manner described in Marburg et al. (ibid). The conjugates are stable polysaccharide-protein conjugates, coupled through bigeneric spacers containing a thioether group and primary amine, which form hydrolytically-labile covalent bonds with the polysaccharide and the protein. Exemplary conjugates are those which may be represented by the formulae, Ps-A-E-S-B-Pro or Ps-A'-S-E'-B'-Pro, wherein Ps represents a polysaccharide; Pro represents a bacterial protein; and A-E-S-B and A'-S-E'-B' constitute bigeneric spaces which contain hydrolytically-stable covalent thioether bonds, and which form covalent bonds (such as hydrolytically-labile ester or amide bonds) with the macromolecules, Pro and Ps. The specific definitions of A,E,S,B,A′,E′ and B′ are presented in Marburg et al. Procedures for preparing polysaccharides and proteins for conjugation, performing conjugation, and determining conjugation are described in the patent.

## Claims

**1.** A method for removing endotoxin from a Gram-negative bacteria fermentation product which comprises the steps of:

(a) growing Gram-negative bacteria in fermentation broth, releasing polysaccharide into the broth, and adding alcohol to the broth to remove impurities by precipitation;
(b) isolating the remaining high molecular weight species and dissolving them in calcium chloride solution, removing additional impurities by completing a minimum of one additional alcohol fractionation, collecting and drying the second alcohol precipitate, dissolving the dry powder thereby obtained in sodium acetate and extracting with phenol to remove impurities, and subsequently removing the phenol from the agueous solution containing polysaccharide by diafiltration with water;
(c) centrifuging remaining high molecular weight species and resolubilizing in a divalent counter ion solution; and
(d) adding alcohol to the solution, cooling the solution, and thereafter incrementally adding alcohol to achieve lipopolysaccharide/polysaccharide precipitation;
characterised in that it also comprises the following step:
(e) mixing lipopolysaccharide and polysaccharide-containing material resulting from the alcohol addition with a nonionic resin, a detergent and a chelating agent under basic pH to remove lipopolysaccharide.

**2.** The method of Claim 1 wherein the initial addition of alcohol and temperature after cooling in step (d) results in an alcohol concentration which is up to 2% below the alcohol concentration at the cloud point, defined as the percentage of alcohol when endotoxin and polysaccharide start to precipitate, causing turbidity.

**3.** The method of Claim 1 wherein the polysaccharide is polyribosylribitol phosphate.

**4.** The method of Claim 1 wherein the chelating agent is sodium citrate.

**5.** The method of Claim 1 wherein the detergent is deoxycholate.

**6.** The method of Claim 1 wherein the resin is a highly porous styrene and divinylbenzene copolymer.

**7.** A method for removing endotoxins from the outer cell of Gram-negative bacteria which comprises the steps of:
(a) growing Gram-negative bacteria in fermentation broth, releasing polysaccharide into the broth, and adding alcohol to remove impurities by precipitation;
(b) isolating the remaining high molecular weight species and dissolving them in calcium chloride solution, removing additional impurities by completing a minimum of one additional alcohol fractionation, collecting and drying the second alcohol precipitate, dissolving the dry powder thereby obtained in sodium acetate and extracting with phenol to remove impurities, and subsequently removing the phenol from the aqueous solution containing polysaccharide by diafiltration with water; and
(c) precipitating and removing impurities by padding ethanol to the product of (b);
characterised in that it also comprises the following steps:
(d) drying the resulting solution of (c), and dissolving the resulting powder in a solution of chelating agent and detergent under basic pH and mixing nonionic resin under suitable conditions; and
(e) removing the resin, chelating agent and detergent and precipitating the polysaccharide from solution with ethanol, centrifuging the precipitate, triturating the pellet with ethanol, and drying the resulting product to form a powder.

**8.** The method of Claim 7 wherein the polysaccharide is polyribosylribitol phosphate.

**9.** The method of Claim 7 wherein the chelating agent is sodium citrate.

**10.** The method of Claim 7 wherein the detergent is sodium deoxycholate.

**11.** The method of Claim 7 wherein the resin is a highly porous styrene and divinylbenzene copolymer.

**12.** The method of Claim 2 wherein the alcohol concentration resulting from the initial addition of alcohol is between about 0.5 and 1% below the alcohol concentration at the cloud point, as defined in claim 2, for that temperature.

**13.** The method of Claim 12, wherein the concentration of alcohol is repeatedly increased by about 0.2% maintaining constant temperature until the cloud point, as defined in claim 2, is reached.

**14.** A method of Claim 13, wherein precipitated material is removed after the cloud point, as defined in claim 2, has been reached, and polysaccharide remaining in solution is dried.

**Patentansprüche**

**1.** Verfahren zur Entfernung von Endotoxin aus einem Fermentationsprodukt gramnegativer Bakterien, welches die Stufen umfaßt:

(a) Züchten von gramnegativen Bakterien in einem Fermentationsmedium, Freisetzen von Polysaccharid in das Medium und Zugeben von Alkohol zu dem Medium, um Verunreinigungen durch Ausfällen zu entfernen;

(b) Isolieren der verbleibenden hochmolekularen Spezies und ihr Auflösen in Calciumchloridlösung, Entfernen weiterer Verunreinigungen, indem mindestens eine weitere Alkoholfraktionierung durchgeführt wird, Sammeln und Trocknen des zweiten Alkoholniederschlags, Auflösen des so erhaltenen, trockenen Pulvers in Natriumacetat und Extrahieren mit Phenol, um Verunreinigungen zu entfernen, und schließlich Entfernen von Phenol aus der wäßrigen Lösung, die Polysaccharid enthält, durch Diafiltration mit Wasser;

(c) Zentrifugieren der restlichen hochmolekularen Spezies und wiederauflösen in einer Losung zweiwertiger Gegenionen; und

(d) Zugeben von Alkohol zu der Lösung, Abkühlen der Lösung und danach schrittweises Zugeben von Alkohol, um eine Fällung von Lipopolysaccharid/Polysaccharid zu erzielen;

dadurch gekennzeichnet, daß es ferner die folgende Stufe umfaßt:

(e) Vermischen des lipopolysaccharid- und polysaccharidhaltigen Materials, das aus der Alkoholzugabe stammt, mit einem nichtionischen Harz, einem Detergens und einem Chelatbildner bei einem basischen pH-Wert, um Lipopolysaccharid zu entfernen.

**2.** Verfahren nach Anspruch 1, bei dem die anfängliche Zugabe des Alkohols und die Temperatur nach dem Abkühlen in Stufe (d) zu einer Alkoholkonzentration führen, die bis zu 2 % unter der Alkoholkonzentration am Trübungspunkt liegt, die als der Prozentsatz von Alkohol definiert ist, bei dem Endotoxin und Polysaccharid auszufallen beginnen und eine Trübung verursachen.

**3.** Verfahren nach Anspruch 1, bei dem das Polysaccharid Polyribosylribitolphosphat ist.

**4.** Verfahren nach Anspruch 1, bei dem der Chelatbildner Natriumcitrat ist.

**5.** Verfahren nach Anspruch 1, bei dem das Detergens Desoxycholat ist.

**6.** Verfahren nach Anspruch 1, bei dem das Harz ein hochporöses Styrol- und Divinylbenzol-Copolymer ist.

**7.** Verfahren zur Entfernung von Endotoxinen aus der äußeren Zelle gramnegativer Bakterien, das die Stufen umfaßt:

(a) Züchten von gramnegativen Bakterien in einem Fermentationsmedium, Freisetzen von Polysaccharid in das Medium und Zugeben von Alkohol zu dem Medium, um Verunreinigungen durch Ausfällen zu entfernen;

(b) Isolieren der verbleibenden hochmolekularen Spezies und ihr Auflösen in Calciumchloridlösung, Entfernen weiterer Verunreinigungen, indem mindestens eine weitere Alkoholfraktionierung durchgeführt wird, Sammeln und Trocknen des zweiten Alkoholniederschlags, Auflösen des so erhaltenen, trockenen Pulvers in Natriumacetat und Extrahieren mit Phenol, um Verunreinigungen zu entfernen, und schließlich Entfernen von Phenol aus der wäßrigen Lösung, die Polysaccharid enthält, durch Diafiltration mit Wasser; und

(c) Ausfällen und Entfernen von Verunreinigungen durch Zugabe von Ethanol zu dem Produkt aus (b);

dadurch gekennzeichnet, daß es ferner die folgenden Stufen umfaßt:

(d) Trocknen der resultierenden Lösung aus (c) und Auflösen des resultierenden Pulvers in einer Lösung des Chelatbildners und des Detergens bei einem basischen pH-Wert und Vermischen des

nichtionischen Harzes unter geeigneten Bedingungen; und
(e) Entfernen des Harzes, des Chelatbildners und des Detergens und Ausfällen des Polysaccharids aus der Lösung mit Ethanol, Zentrifugieren des Niederschlags, Verreiben des Pellets mit Ethanol und Trocknen des resultierenden Produkts unter Bildung eines Pulvers.

**8.** Verfahren nach Anspruch 7, bei dem das Polysaccharid Polyribosylribitolphosphat ist.

**9.** Verfahren nach Anspruch 7, bei dem der Chelatbildner Natriumcitrat ist.

**10.** Verfahren nach Anspruch 7, bei dem das Detergens Natriumdesoxycholat ist.

**11.** Verfahren nach Anspruch 7, bei dem das Harz ein hochporöses Styrol- und Divinylbenzol-Copolymer ist.

**12.** Verfahren nach Anspruch 2, bei dem die Alkoholkonzentration, die sich aus der anfänglichen Zugabe von Alkohol ergibt, zwischen etwa 0,5 und 1 % unterhalb der Alkoholkonzentration des Trübungspunktes, wie in Anspruch 2 definiert, für diese Temperatur liegt.

**13.** Verfahren nach Anspruch 12, bei dem die Alkoholkonzentration wiederholt um 0,2 % vergrößert wird, wobei die Temperatur konstant gehalten wird, bis der Trübungspunkt erreicht ist, wie in Anspruch 2 definiert.

**14.** Verfahren nach Anspruch 13, bei dem das ausgefallene Material, nachdem der Trübungspunkt erreicht ist, entfernt wird, wie in Anspruch 2 definiert ist, und das Polysaccharid, das in der Lösung verbleibt, getrocknet wird.

**Revendications**

**1.** Procédé d'élimination d'endotoxine d'un produit de fermentation de bactéries gram-négatives, qui comprend les étapes de :
(a) culture de bactéries gram-négatives dans un bouillon de fermentation, libération du polysaccharide dans le bouillon, et addition d'alcool au bouillon pour éliminer les impuretés par précipitation;
(b) isolement des espèces de haut poids moléculaire restantes et leur dissolution dans une solution de chlorure de calcium, élimination des impuretés supplémentaires en effectuant au minimum un fractionnement à l'alcool supplémentaire, récupération et séchage du second précipité à l'alcool, dissolution de la poudre sèche ainsi obtenue dans de l'acétate de sodium et extraction au phénol pour éliminer les impuretés, et ensuite élimination du phénol de la solution aqueuse contenant un polysaccharide par diafiltration avec de l'eau,
(c) centrifugation des espèces de haut poids moléculaire restantes et resolubilisation dans une solution de contre-ions divalents; et
(d) addition d'alcool à la solution, refroidissement de la solution et ensuite addition progressive d'alcool pour réaliser une précipitation du lipopolysaccharide/polysaccharide;
caractérisé en ce qu'il comprend également l'étape suivante:
(e) mélange du matériau contenant le lipopolysaccharide et le polysaccharide résultant de l'addition d'alcool avec une résine non ionique, un détergent et un agent chélatant, en pH basique, pour éliminer le lipopolysaccharide.

**2.** Procédé de la revendication 1, dans lequel l'addition d'alcool initiale et la température après refroidissement dans l'étape (d) conduit à une concentration en alcool qui va jusqu'à 2 % en dessous de la concentration en alcool au point d'opacification, défini comme le pourcentage d'alcool auquel l'endotoxine et le polysaccharide commencent à précipiter, causant une turbidité.

**3.** Procédé de la revendication 1, dans lequel le polysaccharide est du phosphate de polyribosylribitol.

**4.** Procédé de la revendication 1, dans lequel l'agent chélatant est du citrate de sodium.

**5.** Procédé de la revendication 1, dans lequel le détergent est du désoxycholate.

6. Procédé de la revendication 1, dans lequel la résine est un copolymère de styrène et de divinylbenzène hautement poreux.

7. Procédé d'élimination des endotoxines de la cellule externe de bactéries gram-négative qui comprend les étapes de:
(a) culture de bactéries gram-négatives dans un bouillon de fermentation, libération du polysaccharide dans le bouillon, et addition d'alcool au bouillon pour éliminer les impuretés par précipitation
(b) isolement des espèces de haut poids moléculaire restantes et leur dissolution dans une solution de chlorure de calcium, élimination des impuretés supplémentaires en effectuant au minimum un fractionnement à l'alcool supplémentaire, récupération et séchage du second précipité à l'alcool, dissolution de la poudre sèche ainsi obtenue dans de l'acétate de sodium et extraction au phénol pour éliminer les impuretés, et ensuite élimination du phénol à partir de la solution aqueuse contenant un polysaccharide par diafiltration avec de l'eau; et
(c) précipitation et élimination des impuretés par addition d'éthanol au produit de (b) ;
caractérisé en ce qu'il comprend aussi les étapes suivantes :
(d) séchage de la solution résultant de (c), et dissolution de la poudre résultante dans une solution d'agent chélatant et de détergent en pH basique et mélange avec une résine non ionique dans des conditions appropriées ; et
(e) élimination de la résine, de l'agent chélatant et du détergent et précipitation du polysaccharide de la solution par de l'éthanol, centrifugation du précipité, trituration du culot avec de l'éthanol, et séchage du produit résultant pour former une poudre.

8. Procédé de la revendication 7, dans lequel le polysaccharide est du phosphate de polyribosylribitol.

9. Procédé de la revendication 7, dans lequel l'agent chélatant est du citrate de sodium

10. Procédé de la revendication 7, dans lequel le détergent est du désoxycholate de sodium.

11. Procédé de la revendication 7, dans lequel la résine est un copolymère de styrène et de divinylbenzène hautement poreux.

12. Procédé de la revendication 2, dans lequel la concentration en alcool résultant de l'addition initiale d'alcool est comprise entre 0,5 et 1 % en dessous de la concentration en alcool au point d'opacification comme défini dans la revendication 2, pour cette température.

13. Procédé de la revendication 1 2, dans lequel la concentration en alcool est augmentée de façon répétée d'environ 0,2 % en maintenant constante la température, jusqu'à ce que le point d'opacification, tel que défini dans la revendication 2, soit atteint.

14. Procédé de la revendication 13, dans lequel le matériau précipité est éliminé après que le point d'opacification, tel que défini dans la revendication, ait été atteint, et le polysaccharide restant dans la solution est séché.